(19) Europäisches Patentamt
European Patent Office
Office européen des brevets

(11) **EP 2 890 340 B1**

(12) **EUROPEAN PATENT SPECIFICATION**

(45) Date of publication and mention
of the grant of the patent:
**01.02.2017 Bulletin 2017/05**

(51) Int Cl.:
***A61F 9/008*** *(2006.01)*

(21) Application number: **12756364.1**

(22) Date of filing: **28.08.2012**

(86) International application number:
**PCT/EP2012/003615**

(87) International publication number:
**WO 2014/032678 (06.03.2014 Gazette 2014/10)**

(54) **SCANNING SYSTEMS TO REDUCE OPAQUE BUBBLE LAYERS**

ABTASTSYSTEME ZUR VERMEIDUNG OPAKER BLASENSCHICHTEN

SYSTÈMES DE BALAYAGE DESTINÉS À RÉDUIRE DES COUCHES DE BULLES OPAQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**08.07.2015 Bulletin 2015/28**

(73) Proprietor: **WaveLight GmbH**
**91058 Erlangen (DE)**

(72) Inventors:
• **SCHUMACHER, Silvia**
**30171 Hannover (DE)**
• **MROCHEN, Michael**
**CH-8193 Eglisau (CH)**

• **WUELLNER, Christian**
**91094 Braeuningshof (DE)**
• **DONITZKY, Christof**
**90542 Eschenau (DE)**
• **VOGLER, Klaus**
**99444 Blankenhain (DE)**

(74) Representative: **Katérle, Axel**
**Wuesthoff & Wuesthoff**
**Patentanwälte PartG mbB**
**Schweigerstraße 2**
**81541 München (DE)**

(56) References cited:
**US-A1- 2003 229 339     US-A1- 2008 212 623**
**US-A1- 2008 243 109     US-A1- 2011 206 071**

EP 2 890 340 B1

**EP 2 890 340 B1**

**Description**

TECHNICAL FIELD

The present disclosure relates generally to surgical devices, and more particularly to scanning systems to reduce opaque bubble layers.

BACKGROUND

In laser surgery, biological tissue may be modified (e.g., ablated, cut, or separated) by inducing a laser-induced optical breakdown (LIOB) in the tissue. The LIOB may yield a small gas volume, or gas bubble, as a residual product, and individual gas bubbles may combine to create a gas-filled cavity. The cavity may cause stresses in and possibly deform the tissue, which may change the transparency or other optical properties of the tissue, yielding an opaque bubble layer (OBL).

In some situations, surgery may need to be halted until the gas diffuses into the tissue, which may take 10 to 30 minutes, as the gas may cause certain problems. For example, the gas may inhibit an eye-tracking device that tracks the position of the eye. As another example, the gas bubbles may penetrate deeper than the actual cutting depth, which may affect subsequent cutting.

Certain known techniques attempt to reduce the effect of the gas bubbles. One technique cuts a channel that carries the gas to the surface of the tissue. Another technique creates a pocket into which the gas can flow. In the system described in the US Patent Application 2011/206071 A1 the laser parameters are adjusted to obtain a tissue region adjusted bubble density.

BRIEF SUMMARY

In certain embodiments, reducing opaque bubble layers (OBLs) comprises receiving information describing a tissue region of a tissue where laser pulses are applied to yield laser-induced optical breakdowns (LIOBs) in the tissue region. The LIOBs yield bubbles of gas. A concentration of the gas in the tissue region is estimated from the information by: calculating a previous concentration of the gas due to one or more previous laser pulses of the laser pulses; calculating a diffusion of the gas away from the tissue region; and estimating the concentration of the gas from the previous concentration of the gas and the diffusion of the gas away from the tissue region. One or more laser parameters are adjusted in response to the concentration of the gas to satisfy a critical concentration rule.

BRIEF DESCRIPTION OF THE DRAWINGS

Exemplary embodiments of the present disclosure will now be described by way of example in greater detail with reference to the attached figures, in which:

FIGURE 1    illustrates an example of a device configured to reduce opaque bubble layers (OBLs) that form during laser surgery according to certain embodiments;

FIGURE 2    illustrates a bubble of gas dissolving into a tissue;

FIGURE 3    illustrates an example of a method that may be used to reduce opaque bubble layers (OBLs) according to certain embodiments;

FIGURE 4    illustrates an example of a pulse line of bubbles; and

FIGURE 5    illustrates an example of diffusion and bridges.

DESCRIPTION OF EXAMPLE EMBODIMENTS

Referring now to the description and drawings, example embodiments of the disclosed apparatuses, systems, and methods are shown in detail. The description and drawings are not intended to be exhaustive or otherwise limit or restrict the claims to the specific embodiments shown in the drawings and disclosed in the description. Although the drawings represent possible embodiments, the drawings are not necessarily to scale and certain features may be simplified, exaggerated, removed, or partially sectioned to better illustrate the embodiments. In addition, certain drawings may be in schematic form.

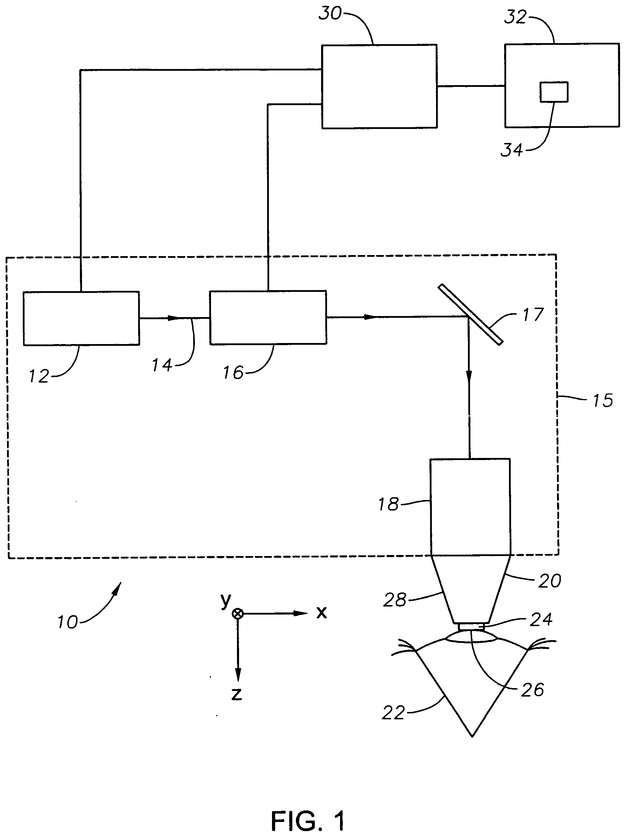

FIGURE 1 illustrates an example of a device 10 configured to reduce opaque bubble layers (OBLs) that form during laser surgery according to certain embodiments. In the embodiments, the device 10 includes a laser device and a control computer. The control computer can receive information describing a tissue region of a tissue where laser pulses are applied by the laser device to yield laser-induced optical breakdowns (LIOBs) in the tissue region. The control computer can estimate a concentration of the gas in the tissue region using this information and adjust one or more laser parameters in response to the concentration of gas to satisfy a critical concentration rule. Adjusting the parameters in this manner may reduce opaque bubble layers (OBLs). In other embodiments, the device 10 may receive laser instructions that include laser parameters that were determined in such manner.

In the illustrated example of FIGURE 1, the device 10 performs surgery on the tissue of an eye 22. The device 10 includes a laser device 15, a patient adapter 20, a control computer 30, and a memory 32 coupled as exemplary shown. The laser device 15 may include a laser source 12, a scanner 16, one or more optical elements 17, and/or a focusing objective 18 coupled as exemplary shown. The patient adapter 20 may include a contact element 24 (which has an abutment face 26 disposed outwardly from a sample) and a sleeve 28 coupled as shown. The memory 32 stores a control program 34. The eye 22 may be biological tissue, such as eye tissue, e.g., corneal tissue.

The laser source 12 generates a laser beam 14 with ultrashort pulses. In this document, an "ultrashort" pulse of light refers to a light pulse that has a duration that is less than a nanosecond, such as on the order of a nanosecond, picosecond, femtosecond, or attosecond or less. The focal point of the laser beam 14 may create a laser-induced optical breakdown (LIOB) in tissues such as the cornea. The laser beam 14 may be precisely focused to allow for precise incisions in the corneal cell layers, which may reduce or avoid unnecessary destruction of other tissue.

Examples of laser source 12 include nanosecond, picosecond, femtosecond, and attosecond lasers. The laser beam 14 may have any suitable wavelength, such as a wavelength in the range of 300 to 1500 nanometers (nm), for example, a wavelength in the range of 300 to 650, 650 to 1050, 1050 to 1250, or 1100 to 1500 nm. The laser beam 14 may also have a relatively small focus volume, e.g., 20 micrometers ($\mu$m) or less, such as 10 $\mu$m or 5 $\mu$m or less, in diameter. In certain embodiments, the laser source 12 and/or delivery channel may be in a vacuum or near vacuum, e.g. less than 100 mbar.

The scanner 16, optical elements 17, and focusing objective 18 are in the beam path. The scanner 16 transversely and longitudinally controls the focal point of the laser beam 14. "Transverse" refers to a direction at right angles to the direction of propagation of the laser beam 14, and "longitudinal" refers to the direction of beam propagation. The transverse plane may be designated as the x-y plane, and the longitudinal direction may be designated as the z-direction. In certain embodiments, the abutment face 26 of the patient adapter 20 is on an x-y plane.

The scanner 16 may transversely direct the laser beam 14 in any suitable manner. For example, the scanner 16 may include a pair of galvanometrically actuated scanner mirrors that can be tilted about mutually perpendicular axes. As another example, the scanner 16 may include an electro-optical crystal that can electro-optically steer the laser beam 14. The scanner 16 may longitudinally direct the laser beam 14 in any suitable manner. For example, the scanner 16 may include a longitudinally adjustable lens, a lens of variable refractive power, or a deformable mirror that can control the z-position of the beam focus. The focus control components of the scanner 16 may be arranged in any suitable manner along the beam path, e.g., in the same or different modular units.

One or more optical elements 17 direct the laser beam 14 towards the focusing objective 18. An optical element 17 may be any suitable optical element that can reflect, refract, and/or diffract the laser beam 14. For example, an optical element 17 may be an immovable deviating mirror. The focusing objective 18 focuses the laser beam 14 onto the patient adapter 20, and may be separably coupled to the patient adapter 20. The focusing objective 18 may be any suitable optical element that can focus the laser radiation, such as an f-theta objective.

Patient adapter 20 interfaces with the cornea of the eye 22. In the example, the patient adapter 20 has a sleeve 28 coupled to a contact element 24. The sleeve 28 couples to the focusing objective 18. The contact element 24 may be translucent or transparent to the laser radiation and has an abutment face 26 that interfaces with the cornea of an eye 22 and may level a portion of the cornea. In certain embodiments, the abutment face 26 is planar and forms a planar area on the cornea. The abutment face 26 may be on an x-y plane, so the planar area is also on an x-y plane. In other embodiments, the abutment face 26 need not be planar, e.g., may be convex or concave.

The control computer 30 controls controllable components, e.g., the laser source 12, scanner 16, and one or more optical elements, in accordance with the control program 34. The control program 34 contains computer code that instructs the controllable components to focus the pulsed laser radiation at a region of the cornea of an eye 22 to photodisrupt at least a portion of the region.

In certain examples of operation, the scanner 16 may direct the laser beam 14 to form incisions of any suitable geometry. Examples of types of incisions include planar incisions and lateral incisions. A planar incision is two-dimensional incision that is typically on an x-y plane. The scanner 16 may form a planar incision by focusing the laser beam 14 at a constant z-value under the abutment face 26 and moving the focus in a pattern in an x-y plane. A lateral incision is an incision that extends from under the corneal surface (such as from a planar incision) to the surface. The scanner 16 may form a lateral incision by changing the z-value of the focus of the laser beam 14 and optionally changing the x and/or y values.

Any suitable portion of the cornea may be photodisrupted. One or more of any of the corneal layers may be selected for photodisruption. In addition, a portion of a cell layer may be photodisrupted in the z-direction, but part of the cell layer may remain on the cornea of an eye 22. Moreover, a particular area (or "target zone") in the x-y plane may be selected for photodisruption. For example, a target zone that forms a planar incision may be photodisrupted.

The device 10 may photodisrupt a corneal layer in any suitable manner. In certain embodiments, the control computer 30 may instruct the laser device to focus the laser beam 14 at a constant z-value under the abutment face 26 and move in a pattern in the x-y plane that substantially covers the target zone. Any suitable pattern may be used. For example, according to a meander pattern or line pattern, the scan path has a constant y-value and moves in the +x direction. When the scan path reaches a point of the border of the target zone, the path moves to a next y value that is a predetermined distance from the previous y-value and then moves in the -x direction until it reaches another point of the border. The scan path continues until the entire target zone is scanned. As another example, according to a spiral pattern, the scan path starts at or near the center of the target zone and moves, e.g., in a spiral pattern or concentric circular pattern until the path reaches the border of the target zone, or vice-versa.

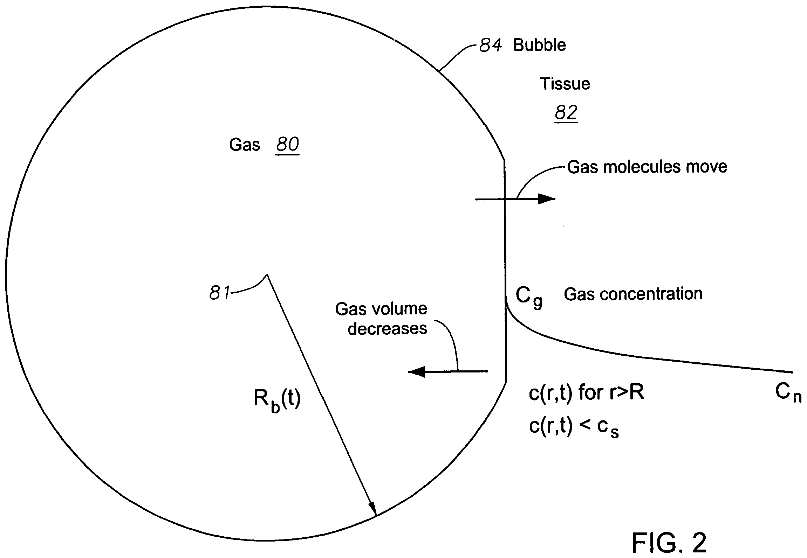

FIGURE 2 illustrates a bubble 84 of gas 80 dissolving into a tissue 82. In certain embodiments, the tissue 82 may be biological tissue, e.g., eye tissue such as corneal tissue, and may include multiple tissue regions. The tissue 82 may include tissue structures and tissue liquid, such as tissue water. The gas 80 may be gas resulting from laser-induced optical breakdown (LIOB) in the tissue 82, and the bubble 84 is a volume of the gas 80. In general, the reduction of bubbles 84 reduces the likelihood of opaque bubble layers (OBLs).

In the illustrated example, the laser device 15 generates a LIOB at the middle point 81 in the tissue 82 and also generates a plasma expansion. The plasma expansion is described as a bubble 84. Gas 80 moves from the bubble 84 to a region of the tissue 82 adjacent to the bubble 84. The dissolved portions of the gas 80 then move away by diffusion. As the gas 80 leaves the bubble 84, the radius (and thus volume) of the bubble 84 decreases. The decrease in radius $R_b(t)$ can be given by:

$$(1) \qquad \frac{\partial}{\partial t} R_b = \frac{D(c_g - c_s)\frac{1}{R_b} - \frac{R_b}{3}\frac{M}{RT}\frac{\partial}{\partial t}P_a}{\frac{M}{RT}\left(P_a + \frac{4\sigma}{3}\frac{1}{R_b}\right)}$$

where D is the diffusion coefficient, $c_g$ is the gas concentration already present in the tissue region, $c_s$ is the saturation coefficient, M is the molar mass of the gas, R is the general gas constant, T is the temperature, $p_a$ is the ambient pressure (e.g., corneal laceration stress), and σ is the surface tension of the gas bubble.

For a single bubble, the solution is:

$$(2) \qquad R_b(t) = -\frac{4\sigma}{3P_a} \pm \sqrt{\left(\frac{4\sigma}{3P_a}\right)^2 - \left(-2\frac{D(c_g - c_s)}{\frac{MP_a}{RT}}t - R_0^2 - 2\left(\frac{4\sigma}{3P_a}\right)R_0\right)}$$

For multiple bubbles, the differential equation can be solved numerically to account for the gas of other bubbles. When smaller bubbles combine to form a larger bubble, the gas dissolving may occur more slowly.

The rate at which the gas bubble 84 dissolves depends on the laser energy, the focus diameter, the repetition rate, as well as the saturation of the gas components in the tissue region and on the volume of the bubble 84. The smaller the saturation, the faster the bubble dissolves, so that decreasing the saturation, particularly the concentration difference D ($c_g$ - $c_s$) reduces the bubbles 84. The saturation can be decreased by diffusion of the components away from the LIOB region, which can be expressed by the diffusion equation:

$$(3) \qquad \frac{\partial c}{\partial t} = D\sigma^2 c$$

where c is the concentration of gas in the region of the bubble. Diffusion may be calculated in any suitable manner and take into account any suitable properties, such as tissue properties and the position and depth of the LIOB in the tissue.

Gas components of one or more previous pulses that have not diffused away from the tissue region contribute to gas saturation at the region, which may affect the diffusion of one or more subsequent pulses. That is, the accumulated gas components of one or more previous pulses may affect the diffusion of one or more subsequent pulses. In certain embodiments, one or more laser parameters that affect the relationship between laser pulses may be adjusted to decrease the effect that previous pulses have on subsequent pulses. By decreasing this effect, bubbles are more likely to dissolve, which may reduce the likelihood of opaque bubble layers.

In some cases, the parameters may be adjusted such that the concentration of gas satisfies a critical concentration rule where the saturation has little or no effect. For example, a critical concentration rule may be a maximum concentration below (or at) which subsequent pulses are not affected in an unsatisfactory manner. A tissue with a concentration at or above (or just above) the critical concentration may be described as supersaturated. A critical concentration may have any suitable value, such as a value in the range of less than 1 kg/m$^3$, depending on the gas and tissue components and the temperature and partial pressure. For reference, the saturation of an air bubble in water is

$$(4) \qquad \frac{Saturation\ of\ concentration}{density} = 0.02.$$

Laser parameters are parameters that instruct the laser to operate in a particular manner, and may designate, e.g., the energy or position (in the x, y, and/or z direction) of a laser pulse or the timing of one or more pulses. An example of a laser parameter that can be adjusted is the repetition rate, including separation parameters that designate the separation between pulses, such as a temporal separation or a spatial separation of a sequence of pulses. A temporal separation of a sequence of pulses is the time elapsed between subsequent pulses, and may be given by a pulse repetition rate. A greater elapsed time allows for gas saturation from previous pulses to decrease. Thus, increasing temporal separation increases the likelihood that the bubbles will be dissolved.

In certain embodiments, a critical temporal separation may designate a minimum temporal separation at which a previous pulse may cause the gas concentration for a subsequent pulse to reach a critical concentration. Accordingly, the actual temporal separation may be selected to be as greater than the critical temporal separation. The critical temporal separation may be derived from the thermal diffusion:

$$(5) \qquad l_{Diff} = 2\sqrt{D\tau}$$

with D= 1.43 x 10$^{-7}$ m$^2$/s and

$$(6) \qquad f = \frac{1}{\tau}$$

may have any suitable value, such as a value in the range of 1 nanosecond (ns) to 1 ms or 1kHz to 1 GHz.

A spatial separation of a sequence of pulses is the distance between subsequent pulses, and may be given by a pulse scan pattern. A greater distance decreases the effect that gas saturation from previous pulses has an impact on subsequent pulses. Thus, increasing spatial separation increases the likelihood that the bubbles will dissolve and do not interfere with the next pulse in a neighborhood location. In certain embodiments, a critical spatial separation may designate a minimum spatial separation at which one pulse may cause the gas concentration for another pulse to reach a critical concentration. Accordingly, the actual spatial separation may be selected to be greater than the critical spatial separation. The critical spatial separation may have any suitable value, such as a value in the range of 0.1 $\mu$m to 20 mm, such as 0.1 $\mu$m to 10 $\mu$m.

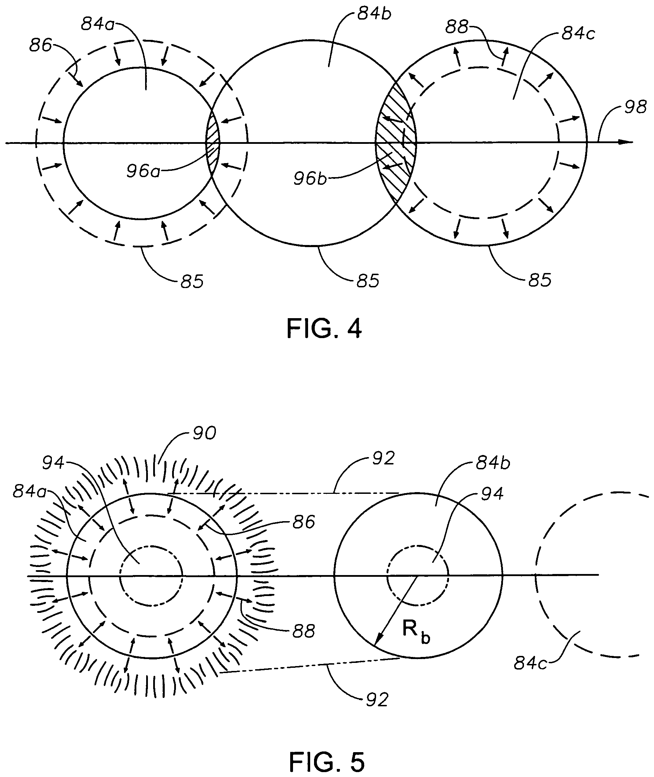

FIGURE 4 illustrates an example of a pulse line 98 of bubbles 84 (84a, 84b, 84c). The pulse line 98 is a sequence of laser pulses with a defined repetition rate. In the example, the bubble 84a is decreasing (as shown by arrows 86) after full expansion 85. The bubble 84b is at full expansion 85. The bubble 84c is increasing (as shown by arrows 88) to full expansion 85. Intersection zones 96a and 96b are regions where bubbles 84a and 84b overlap and bubbles 84b and 84c overlap, respectively. The zones 96 change over time. Intersections of bubbles 84 of successive pulses should be avoided because bubbles 84 can unify to yield an opaque bubble layer. Thus, a substantial portion of a subsequent bubble should be located outside of the space of a preceding bubble to avoid bubble unification.

The radius of a bubble that is generated instantaneously may be expressed as:

$$(7) \qquad \tau_L < \tau_o \ll f^{-1}$$

where f represents the repetition rate in [s$^{-1}$].

FIGURE 5 illustrates the example of FIGURE 4 in more detail. The diffusion 90 into the surrounding tissue 82 occurs when the bubble 84 starts decreasing from full expansion 85. A bridge 92 appears if the distance between the bubbles 84 is too small. This situation should be avoided, as it may yield large OBLs.

In certain embodiments, parameters for the pulse line 98 are selected such that a subsequent pulse does not hit an area 94 that has been affected by a preceding pulse. The area 94 may have been affected by thermal destructive impact or may have experienced another change. In some embodiments, the next focus location may be placed outside of the area 94.

Bubbles 84 may be separated using any suitable parameter. Examples of parameters include, the spot separation, which is may be expressed as:

$$(8) \qquad d_{Spot} \geq 2r_{bubble} + 2r_{diffusive\,[gas]}$$

the bubble radius $R_b$, which may be expressed as:

$$(9) \qquad r_{bubble} = \text{function of } (E_L, \tau_L) \text{ or } R_o \sim \sqrt[3]{E_L}$$

or the laser pulse duration, which may be expressed as:

$$(10) \qquad \tau_L = \text{function of } (D, c_g\text{-}c_s), r_{bubble}),$$

where $E_L$ represents the laser energy and $c_g\text{-}c_s$ represents the concentration gradient.

Spatial and temporal separation may be determined in any suitable manner. The spot separation may be expressed as:

$$(11) \qquad d_{Spot} \geq l_{Diff}(t) \approx \sqrt{D} \; \Delta t \geq \sqrt{D \frac{1}{f}}$$

or, for a given repetition rate:

$$(12) \qquad d_{Spot\,[\mu m]} > \sim 12 \, (f_{[kHz]})^{-1/2}$$

where $l_{Diff}$ represents the thermal diffusion length, $\Delta t = f^{-1}$ represents the time interval between two pulses, t represents time, and D represents the thermal diffusivity of the cornea $1.43 \times 10^{-7}$ m$^2$/s.

The pulse repetition rate may be adjusted to prevent a subsequent laser pulse from striking an area 94 that has been affected by the thermal distortions of a preceding pulse.

$$(13) \qquad f \geq \frac{D}{d_{Spot}^2}$$

or as:

$$(14) \qquad f_{[kHz]} > \sim 144 \, d^{-2}_{[\mu m]}$$

For example, if the spot separation of the focuses is 1 $\mu$m, the repetition rate may be greater than 144 kHz.

Any suitable event related to OBLs may trigger the adjustment of laser parameters. In certain embodiments, one or more sensors can take measurements used to calculate the values discussed above. In addition or in the alternative, an imaging device, e.g., a camera and/or optical coherence tomography (OCT) system, may detect OBLs, and the system may automatically adjust the parameters.

Although the embodiments use separation parameters, any other suitable parameters may be adjusted to reduce the likelihood of reaching a critical condition. For example, the pulse energy, cutting depth, or other suitable parameter may be adjusted.

One or more laser parameters may be adjusted in any suitable manner. For example, if a separation parameter yields gas concentrations greater than the critical concentration, the value of the parameter may be decreased. In certain embodiments, laser parameters may be adjusted in coordination with each other. For example, if a combination of a temporal separation and a spatial separation yields a satisfactory gas concentration, decreasing the temporal separation and increasing the spatial separation or decreasing the spatial separation and increasing the temporal separation may still yield a satisfactory gas concentration.

Laser parameters, such as the pulse repetition rate, may be selected for pulses in any suitable manner. In certain embodiments, the parameters may be selected such that the critical concentration rule is satisfied throughout the tissue to reduce or substantially eliminate the occurrence of opaque bubble layers. In certain embodiments, the parameters may be selected such that the critical concentration rule is satisfied in certain tissue regions to reduce or substantially eliminate the occurrence of opaque bubble layers in those regions, but not satisfied in other tissue regions to allow for the occurrence of opaque bubble layers in the other regions. In these embodiments, opaque bubble layers may be formed in regions where the layers will not likely negatively impact the surgery, e.g., peripheral in the deep stroma of the cornea.

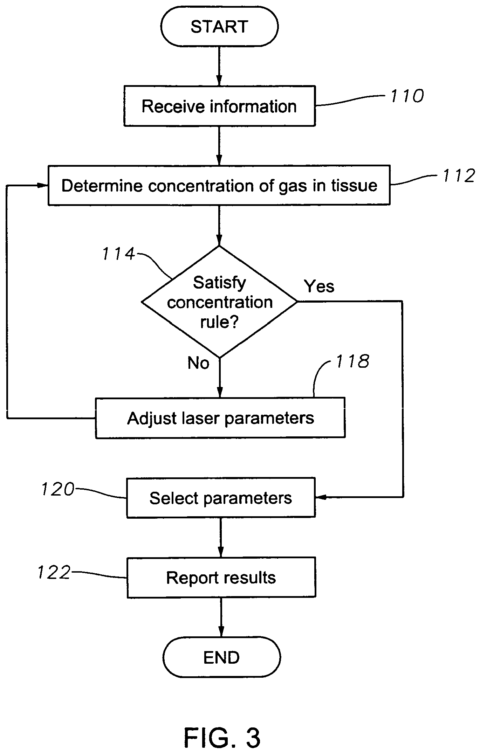

FIGURE 3 illustrates an example of a method that may be used to reduce opaque bubble layers (OBLs) that can form during laser surgery. The method may be performed by any suitable computing system, such as the control computer 30 or other computing system.

The method starts at step 110, where the computing system receives information describing a tissue region of a tissue where laser pulses are applied to yield laser-induced optical breakdowns (LIOBs) in the tissue region. The gas concentration in the tissue is estimated from the information at step 112. The gas concentration may be estimated in any suitable manner. In certain embodiments, the gas concentration is calculated. In the embodiments, a previous concentration of the gas due to one or more previous laser pulses is calculated. A diffusion of the gas away from the tissue region is calculated. The concentration of the gas is estimated using the previous concentration of the gas and the diffusion of the gas away from the tissue region.

In other embodiments, the gas concentration is calculated using a simulation. In the embodiments, a simulation of the laser-induced optical breakdowns in the tissue region is performed and the concentration of the gas is estimated from the simulation. In other embodiments, the concentration of the gas is measured the tissue region using, e.g., optical oxygen sensor, optical coherence tomography (OCT), or multi-photon imaging.

At step 114, a critical concentration rule designates a maximum concentration below which subsequent pulses are not affected in an unsatisfactory manner. A gas concentration may satisfy the rule if the gas concentration is below (or below or at) the maximum concentration or may fail to satisfy the rule if the gas concentration is at or above (or just above) the maximum concentration. If the gas concentration fails to satisfy the rule, the method proceeds to step 118.

Laser parameters may be adjusted in response to the concentration at step 118. The laser parameters may be adjusted in any suitable manner. For example, a spatial and/or temporal separation of the pulses may be increased. As another example, a spatial (or temporal) separation may be increased, but a temporal (or spatial) separation may be decreased. The method returns to step 112 to estimate the concentration of gas.

If the gas concentration satisfies the rule, the method proceeds to step 120 to select the laser parameters to be used for the pulses. The laser parameters may include the energy of the laser, spot location, and/or laser pulse duration. The laser parameters may be selected in any suitable manner, e.g., or For example, laser parameters of substantially all tissue regions of a tissue volume that satisfy the critical concentration may be selected in order to reduce or substantially eliminate the occurrence of an opaque bubble layer in the tissue volume. As another example, laser parameters of one or more tissue regions of a first portion of a tissue volume that satisfy the critical concentration may be selected to reduce or substantially eliminate the occurrence of an opaque bubble layer in the first portion, and laser parameters of one or more tissue regions of a second portion of the tissue volume that fail to satisfy the critical concentration may be selected in order to allow for the occurrence of an opaque bubble layer in the second portion.

The results are reported at step 122. The results may be reported in any suitable manner, e.g., as a display, printout, or data transfer.

Embodiments of the method may be performed in any suitable application. For example, a method may be performed with a computer simulation to yield laser device instructions that can be used for actual surgery. In the example, the computer simulation receives initial information, which includes initial conditions, and then simulates creation of laser-induced optical breakdowns in a tissue region. The gas concentration may be calculated from the simulations, and parameters may be adjusted if the concentration does not satisfy a concentration rule. The embodiment may be performed iteratively until the concentration satisfies the concentration rule. The laser parameters may be used for laser instructions for similar initial conditions. The laser instructions may be input into a laser system that can perform the actual surgery.

As another example, an embodiment may be performed by a laser system in real time during an actual surgery. In the example, the laser system receives initial information, which includes initial conditions, and then proceeds to create laser-induced optical breakdowns in a tissue region. The gas concentration may be measured, and parameters may be adjusted until the concentration satisfies the concentration rule.

A component of the systems and apparatuses disclosed herein may include an interface, logic, memory, and/or other suitable element, any of which may include hardware and/or software. An interface can receive input, send output, process the input and/or output, and/or perform other suitable operations. Logic can perform the operations of a component, for example, execute instructions to generate output from input. Logic may be encoded in memory and may perform operations when executed by a computer. Logic may be a processor, such as one or more computers, one or more microprocessors, one or more applications, and/or other logic. A memory can store information and may comprise one or more tangible, computer-readable, and/or computer-executable storage medium. Examples of memory include computer memory (for example, Random Access Memory (RAM) or Read Only Memory (ROM)), mass storage media (for example, a hard disk), removable storage media (for example, a Compact Disk (CD) or a Digital Video Disk (DVD)), database and/or network storage (for example, a server), and/or other computer-readable media.

In particular embodiments, operations of the embodiments may be performed by one or more computer readable media encoded with a computer program, software, computer executable instructions, and/or instructions capable of being executed by a computer. In particular embodiments, the operations may be performed by one or more computer readable media storing, embodied with, and/or encoded with a computer program and/or having a stored and/or an encoded computer program.

Although this disclosure has been described in terms of certain embodiments, modifications (such as changes, substitutions, additions, omissions, and/or other modifications) of the embodiments will be apparent to those skilled in the art. Accordingly, modifications may be made to the embodiments without departing from the scope of the invention. For example, modifications may be made to the systems and apparatuses disclosed herein. The components of the systems and apparatuses may be integrated or separated, and the operations of the systems and apparatuses may be performed by more, fewer, or other components. As another example, modifications may be made to the methods disclosed herein. The methods may include more, fewer, or other steps, and the steps may be performed in any suitable order.

Other modifications are possible without departing from the scope of the invention. For example, the description illustrates embodiments in particular practical applications, yet other applications will be apparent to those skilled in the art. In addition, future developments will occur in the arts discussed herein, and the disclosed systems, apparatuses, and methods will be utilized with such future developments.

The scope of the invention should not be determined with reference to the description. In accordance with patent statutes, the description explains and illustrates the principles and modes of operation of the invention using exemplary embodiments. The description enables others skilled in the art to utilize the systems, apparatuses, and methods in various embodiments and with various modifications, but should not be used to determine the scope of the invention.

The scope of the invention should be determined with reference to the claims and the full scope of equivalents to which the claims are entitled. All claims terms should be given their broadest reasonable constructions and their ordinary meanings as understood by those skilled in the art, unless an explicit indication to the contrary is made herein. For example, use of the singular articles such as "a," "the," etc. should be read to recite one or more of the indicated elements, unless a claim recites an explicit limitation to the contrary. As another example, "each" refers to each member of a set or each member of a subset of a set, where a set may include zero, one, or more than one element. In sum, the invention is capable of modification, and the scope of the invention should be determined, not with reference to the description, but with reference to the claims and their full scope of equivalents.

## Claims

1. A system comprising:

   a laser device configured to apply a plurality of laser pulses to a tissue region of a tissue to yield a plurality of laser-induced optical breakdowns (LIOBs) in the tissue region, the LIOBs yielding a plurality of bubbles of gas; and
   a control computer configured to:

      receive information describing the tissue region;
      estimate from the information a concentration of the gas in the tissue region by:

         calculating a previous concentration of the gas due to one or more previous laser pulses of the laser pulses;
         calculating a diffusion of the gas away from the tissue region; and

estimating the concentration of the gas from the previous concentration of the gas and the diffusion of the gas away from the tissue region; and

adjust one or more laser parameters in response to the concentration of the gas to satisfy a critical concentration rule.

2. The system of Claim 1, the estimating from the information the concentration of the gas further comprising:

simulating with a simulation the laser-induced optical breakdowns in the tissue region; and
estimating the concentration of the gas in the tissue region from the simulation.

3. The system of Claim 1 or 2, the estimating from the information the concentration of the gas further comprising:

measuring the concentration of the gas in the tissue region.

4. The system of any of Claims 1 to 3, the adjusting the one or more laser parameters further comprising:

increasing a spatial separation between at least two laser pulses.

5. The system of any of Claims 1 to 4, the adjusting the one or more laser parameters further comprising:

increasing a temporal separation between at least two laser pulses.

6. The system of any of Claims 1 to 5, the adjusting the one or more laser parameters further comprising:

increasing a temporal separation between at least two laser pulses; and
decreasing a spatial separation between the at least two laser pulses.

7. The system of any of Claims 1 to 6, the adjusting the one or more laser parameters further comprising:

increasing a spatial separation between at least two laser pulses; and
decreasing a temporal separation between the at least two laser pulses.

8. The system of any of Claims 1 to 7, the control computer further configured to:

select the one or more laser parameters that satisfy the critical concentration rule in the tissue region and a plurality of other tissue regions of the tissue in order to reduce the occurrence of an opaque bubble layer.

9. The system of any of Claims 1 to 8, the control computer further configured to:

select the one or more laser parameters that satisfy the critical concentration rule in the tissue region in order to reduce the occurrence of an opaque bubble layer; and
select the one or more laser parameters that fail to satisfy the critical concentration rule in a second tissue region of the tissue in order to allow for the occurrence of an opaque bubble layer.

10. The system of any of Claims 1 to 9, the control computer further configured to:

detect the bubbles using an imaging device; and
adjust the one or more laser parameters in response to detecting the bubbles.


**Patentansprüche**

1. System, umfassend:

eine Laservorrichtung, gestaltet zum Aufbringen einer Vielzahl von Laserpulsen auf einen Gewebebereich eines Gewebes, um eine Vielzahl von laserinduzierten optischen Breakdowns (LIOBs) in dem Gewebebereich zu erzeugen, wobei die LIOBs eine Vielzahl von Gasblasen erzeugen; und

einen Steuercomputer, gestaltet zum:

Empfangen von Informationen, die den Gewebebereich beschreiben;
aus den Informationen Abschätzen einer Konzentration des Gases in dem Gewebebereich durch:

Berechnen einer vorherigen Konzentration des Gases durch einen oder mehrere vorhergehende Laserpulse der Laserpulse;
Berechnen einer Diffusion des Gases weg von dem Gewebebereich; und
Abschätzen der Konzentration des Gases aus der vorhergehenden Konzentration des Gases und der Diffusion des Gases weg von dem Gewebebereich; und

Anpassen eines oder mehrerer Laserparameter in Antwort auf die Konzentration des Gases, um eine kritische Konzentrationsbedingung zu erfüllen.

2. System gemäß Anspruch 1, wobei das Abschätzen der Konzentration des Gases aus den Informationen ferner umfasst:

Simulieren mit einer Simulation der laserinduzierten optischen Breakdowns in dem Gewebebereich; und
Abschätzen der Konzentration des Gases in dem Gewebebereich aus der Simulation.

3. System gemäß Anspruch 1 oder 2, wobei das Abschätzen der Konzentration des Gases aus den Informationen ferner umfasst:

Messen der Konzentration des Gases in dem Gewebebereich.

4. System gemäß einem der Ansprüche 1 bis 3, wobei das Anpassen des einen oder der mehreren Laserparameter ferner umfasst:

Erhöhen der räumlichen Trennung zwischen wenigstens zwei Laserpulsen.

5. System gemäß einem der Ansprüche 1 bis 4, wobei das Anpassen des einen oder der mehreren Laserparameter ferner umfasst:

Erhöhen der zeitlichen Trennung zwischen wenigstens zwei Laserpulsen.

6. System gemäß einem der Ansprüche 1 bis 5, wobei das Anpassen des einen oder der mehreren Laserparameter ferner umfasst:

Erhöhen einer zeitlichen Trennung zwischen wenigstens zwei Laserpulsen; und
Verringern einer räumlichen Trennung zwischen den wenigstens zwei Laserpulsen.

7. System gemäß einem der Ansprüche 1 bis 6, wobei das Anpassen des einen oder der mehreren Laserparameter ferner umfasst:

Erhöhen einer räumlichen Trennung zwischen wenigstens zwei Laserpulsen; und
Verringern einer zeitlichen Trennung zwischen den wenigstens zwei Laserpulsen.

8. System gemäß einem der Ansprüche 1 bis 7, wobei der Steuercomputer ferner gestaltet ist zum:

Auswählen des einen oder der mehreren Laserparameter, die die kritische Konzentrationsbedingung in dem Gewebebereich und einer Vielzahl anderer Gewebebereiche des Gewebes erfüllen, um das Auftreten einer undurchsichtigen Blasenschicht zu verringern.

9. System gemäß einem der Ansprüche 1 bis 8, wobei der Steuercomputer ferner gestaltet ist zum:

Auswählen des einen oder der mehreren Laserparameter, die die kritische Konzentrationsbedingung in dem Gewebebereich erfüllen, um das Auftreten einer undurchsichtigen Blasenschicht zu verringern; und
Auswählen des einen oder der mehreren Laserparameter, die die kritische Konzentrationsbedingung in einem zweiten Gewebebereich des Gewebes nicht erfüllen, um das Auftreten einer undurchsichtigen Blasenschicht zu erlauben.

10. System gemäß einem der Ansprüche 1 bis 9, wobei der Steuercomputer ferner gestaltet ist zum:

Nachweisen der Blasen unter Verwendung einer Bildgebungsvorrichtung; und
Anpassen des einen oder der mehrere Laserparameter in Antwort auf das Nachweisen der Blasen.

**Revendications**

1. Système comprenant :

un dispositif laser configuré pour appliquer une pluralité d'impulsions laser à une région tissulaire d'un tissu pour produire une pluralité de ruptures optiques induites par laser (LIOB) dans la région tissulaire, les LIOB conduisant à une pluralité de bulles de gaz ; et
un ordinateur de commande configuré pour :

recevoir des informations décrivant la région tissulaire ;
estimer, à partir des informations, une concentration du gaz dans la région tissulaire ; en
calculant une concentration préalable du gaz due à une ou
plusieurs impulsions laser antérieures des impulsions laser ;
calculant une diffusion du gaz hors de la région tissulaire ;
et
estimant la concentration du gaz à partir de la concentration précédente du gaz et de la diffusion du gaz hors de la région tissulaire ; et
ajuster un ou plusieurs paramètres du laser en réponse à la concentration du gaz pour satisfaire une règle de concentration critique.

2. Système selon la revendication 1, l'estimation, à partir des informations, de la concentration du gaz comprenant en outre les étapes suivantes :

simuler, avec une simulation, les ruptures optiques induites par laser dans la région tissulaire ; et
estimer la concentration du gaz dans la région tissulaire à partir de la simulation.

3. Système selon la revendication 1 ou la revendication 2, l'estimation, à partir des informations, de la concentration du gaz, comprenant en outre l'étape suivante :

mesurer la concentration du gaz dans la région tissulaire.

4. Système selon l'une quelconque des revendications 1 à 3, l'ajustement des un ou plusieurs paramètres du laser comprenant en outre l'étape suivante :

augmenter une séparation spatiale entre au moins deux impulsions laser.

5. Système selon l'une quelconque des revendications 1 à 4, l'ajustement des un ou plusieurs paramètres du laser comprenant en outre l'étape suivante :

augmenter un écart temporel entre au moins deux impulsions laser.

6. Système selon l'une quelconque des revendications 1 à 5, l'ajustement des un ou plusieurs paramètres du laser comprenant en outre les étapes suivantes :

augmenter un écart temporel entre au moins deux impulsions laser ; et
diminuer une séparation spatiale entre les au moins deux impulsions laser.

7. Système selon l'une quelconque des revendications 1 à 6, l'ajustement des un ou plusieurs paramètres du laser comprenant en outre les étapes suivantes :

augmenter une séparation spatiale entre au moins deux impulsions laser ; et
diminuer un écart temporel entre les au moins deux impulsions laser.

8. Système selon l'une quelconque des revendications 1 à 7, l'ordinateur de commande étant en outre configuré pour :

sélectionner les un ou plusieurs paramètres du laser qui satisfont la règle de concentration critique dans la région tissulaire et une pluralité d'autres régions tissulaires du tissu, afin de réduire l'apparition d'une couche de bulles opaques.

9. Système selon l'une quelconque des revendications 1 à 8, l'ordinateur de commande étant en outre configuré pour :

sélectionner les un ou plusieurs paramètres du laser qui satisfont la règle de concentration critique dans la région tissulaire de manière à réduire l'apparition d'une couche de bulles opaques ; et
sélectionner les un ou plusieurs paramètres du laser qui ne satisfont pas la règle de concentration critique dans une seconde région tissulaire du tissu de manière à permettre l'apparition d'une couche de bulles opaques.

10. Système selon l'une quelconque des revendications 1 à 9, l'ordinateur de commande étant en outre configuré pour :

détecter les bulles au moyen d'un dispositif d'imagerie ; et
ajuster les un ou plusieurs paramètres du laser en réponse à la détection des bulles.

**FIG. 1**

FIG. 2

START

Receive information —110

Determine concentration of gas in tissue —112

114— Satisfy concentration rule? — Yes

No

118 Adjust laser parameters

120— Select parameters

122— Report results

END

FIG. 3

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 2011206071 A1